# EUROPÄISCHE PATENTANMELDUNG

(11) **EP 1 830 434 A1**
(43) Veröffentlichungstag der Anmeldung: **05.09.2007**
(21) Anmeldenummer: 07000523.6
(22) Anmeldetag: 11.01.2007
(51) Int. Cl.: H01R 13/46

(54) **Teil einer elektrischen Kuppelvorrichtung**

(30) Priorität: 02.03.2006 DE 102006009733
(71) Anmelder: ODU- Steckverbindungssysteme GmbH & Co. KG, 84453 Mühldorf a. Inn (DE)
(72) Erfinder: Jacobi, Wolfgang, Dr.-Ing., 81925 München (DE)
(74) Vertreter: Leinweber & Zimmermann

(57) **Zusammenfassung**

Teil einer elektrischen Kuppelvorrichtung, insbesondere zum Einsatz in der Medizintechnik, mit einer Anzeigeeinrichtung zum Anzeigen einer aus einer Vorbereitungsmaßnahme für einen Einsatz des Teils resultierenden Einwirkung auf das Teil, wobei die Anzeigeeinrichtung ein Sichtfeld aufweist, dessen farbliches Erscheinungsbild sich als Reaktion auf die aus der Vorbereitungsmaßnahme resultierende Einwirkung zumindest abschnittsweise ändert.

## Beschreibung

Die Erfindung betrifft ein Teil einer elektrischen Kuppelvorrichtung, insbesondere zum Einsatz in der Medizintechnik, mit einer Anzeigeeinrichtung zum Anzeigen einer aus einer Vorbereitungsmaßnahme für einen Einsatz des Teils resultierenden Einwirkung auf das Teil, ein Verfahren zum Herstellen eines solchen Teils sowie die Verwendung eines Stoffes in einem solchen Teil.

Elektrische Kuppelvorrichtungen werden in einer großen Anzahl im medizinischen Bereich eingesetzt, u.a. auch in Operationssälen. Sie stellen ein Kontaminationsrisiko dar. Wie andere bei der Operation verwendeten Gerätschaften, wie beispielsweise das Operationsbesteck oder auch Kleidungsstücke des OP-Teams, können auch die Kuppelvorrichtungen aufgrund des Kontaminationsrisikos nicht ohne weiteres bei einer weiteren Operation wieder eingesetzt werden. Je nach Wert solcher Gerätschaften werden diese nach der Operation daher entweder weggeworfen, oder einer Vorbereitungsmaßnahme ausgesetzt, bei der möglichst alle vorhandenen Keime abgetötet werden. So werden etwa Gummihandschuhe des OP-Teams weggeworfen, während das vergleichsweise wertvolle Operationsbesteck nach erfolgter Vorbereitungsmaßnahme wieder verwendet wird. Bei den Operationen eingesetzte elektrische Kuppelvorrichtungen werden häufig wiederverwendet, weshalb sie Vorbereitungsmaßnahmen vor der nächsten Verwendung unterzogen werden müssen.

Eine weit verbreitete Vorbereitungsmaßnahme ist das Autoklavieren (Dampfsterilisieren). Dabei wird ein zu dekontaminierendes Gerät in einer Autoklave (Dampfdruck-Sterilisator) extremen atmosphärischen Einflüssen wie hohen Temperaturen, hohen aber auch niedrigen Drücken oder hoher Luftfeuchtigkeit ausgesetzt. Auf diese Weise können auch sehr resistente Viren, z. B. Hepatitis B/C oder HIV (Aids) auf zuverlässige Art vernichtet werden.

Durch das Autoklavieren wird die Kuppelvorrichtung allerdings angegriffen. Die Kuppelvorrichtungen können somit nicht unbegrenzt wiederverwendet werden, sondern nur so lange, wie deren sachgerechtes Funktionieren noch sichergestellt ist.

Ist das sachgerechte Funktionieren der elektrischen Kuppelvorrichtung nicht mehr gewährleistet, indem z.B. die Steckverbindung zu locker wird, können gefährliche Situationen auftreten - man denke nur an eine Kontaktunterbrechung inmitten einer Operation, deren Erfolg am seidenen Faden hängt. Um solche gefährlichen Situationen gar nicht erst aufkommen zu lassen, sollten bzw. müssen die Kuppelvorrichtungen nach einem bestimmten Festigkeitsverlust bzw. Verschleiß durch Neuteile ersetzt werden. Um festzustellen, wann ein Ersatz nötig ist, sind herkömmliche Kuppelvorrichtungen mit einer Anzeigeeinrichtung in Form eines Zählers ausgestattet. Konkret wird beispielsweise ein Bimetall verwendet, dessen Komponenten sich aufgrund der beim Autoklavieren auftretenden hohen Temperaturen unterschiedlich ausdehnen. Die unterschiedliche Ausdehnung sorgt dann dafür, daß ein weiter vorgesehenes Zählwerk um 1 vorgerückt wird.

Eine derartige Zähleinrichtung ist allerdings baulich recht aufwendig. Darüber hinaus hat es sich herausgestellt, daß der Zählmechanismus fehleranfällig ist und ggf. funktionsunfähig wird. Dazukommt, daß eine auftretende Funktionstüchtigkeit bei normaler Handhabung gar nicht bemerkt wird, da im Regelfall eine Mehrzahl gleicher oder ähnlicher Kuppelvorrichtungen gleichzeitig autoklaviert wird, und nicht jede einzelne Kuppelvorrichtung auf ihre Anzeige vor und nach eines Zyklus kontrolliert wird. Bei Funktionsuntüchtigkeit ergibt sich darüber hinaus eine Situation, die noch problematischer ist als eine Situation ohne Anzeigeeinrichtung. Denn der Benutzer, z.B. eine OP-Schwester, ist durch die fehlerhafte Anzeige im sicheren Glauben, daß die Kuppelvorrichtung noch problemlos sachgerecht funktioniert, während sie in Wirklichkeit schon ein hohes Ausfallrisiko in sich birgt.

Der Erfindung liegt daher die Aufgabe zugrunde, ein Teil einer elektrischen Kuppelvorrichtung der eingangs genannten Art bereitzustellen, dessen Anzeigeeinrichtung baulich einfach ist und zuverlässig anzeigt.

Zur Lösung dieser Aufgabe weist die Anzeigeeinrichtung ein Sichtfeld auf, dessen farbliches Erscheinungsbild sich als Reaktion auf die aus der Vorbereitungsmaßnahme resultierende Einwirkung zumindest abschnittsweise ändert. Die Anzeigeeinrichtung ist denselben Einwirkungen unterworfen, wie das Teil der Kuppelvorrichtung selbst, und mißt gewisserweise diese Einwirkung(en), indem es sein Aussehen verändert. Die Veränderungen sind zumindest teilweise irreversibel, d.h. sie bilden sich höchstens auf einer Zeitskala zurück, die groß ist im Vergleich zu der üblichen Lebensdauer der elektrischen Kuppelvorrichtung. Wären sie vollständig reversibel, bestünde das Risiko, daß die vom Sichtfeld angezeigte Einwirkung von der tatsächlich erfahrenen Einwirkung abweicht.

Darüber hinaus ist dem an farbliche Erscheinungsbilder gewohnte Benutzer eine einfache und für ihn angenehme Ablesemöglichkeit gegeben. Ein kurzer Blick auf das Sichtfeld gibt ihm die Möglichkeit, festzustellen, ob er das entsprechende Teil der Kuppelvorrichtung noch weiter verwenden darf, oder es aussortieren und durch ein Neuteil ersetzen muß. Dabei ist es allerdings nicht erforderlich, daß er jede beliebig kleine Änderung auf eine beispielsweise sehr geringe Einwirkung (z.B. nur eine durchgeführte Vorbereitungsmaßnahme) erkennt. In einer besonders vorteilhaften Ausführungsform ist daher vorgesehen, daß die Änderung des farblichen Erscheinungsbilds von einem ersten Zustand in einen zweiten Zustand erfolgt, deren Unterschied mit bloßem Auge erkennbar ist, wenn die Einwirkung ein vorbestimmtes Maß übersteigt. Dabei kann das vorbestimmte Maß der Einwirkung einerseits dasjenige Maß sein, bei dem eine Ersetzung durch ein Neuteil erfolgen sollte, was der Benutzer mit einem Blick feststellen kann. Der zweite Zustand kann aber auch ein Zwischenzustand sein, der dem Benutzer eine Zwischeninformation anzeigt, wie etwa, daß das Teil bereits der Hälfte einer maximal vorgesehenen Einwirkung ausgesetzt worden ist.

Zweckmäßig ist vorgesehen, daß in dem ersten und in dem zweiten Zustand unterschiedliche Farbtöne erscheinen. So können beispielsweise Farbtöne mit Signalwirkung z.B. Grün, Gelb oder Rot erscheinen, die dem Benutzer in einfachster symbolischer Weise Informationen bereitstellen. Es wäre aber auch denkbar, daß ein Farbton abgeschwächt wird und in einer anderen Farbstufe erscheint.

Auch kann es von Vorteil sein, daß in dem ersten und in dem zweiten Zustand unterschiedliche Graustufen erscheinen. So wird ein ausreichendes Unterscheidungskriterium gegeben, was besonders einfach und kostengünstig hergestellt werden kann.

Es ist von Vorteil, wenn sich das vorbestimmte Maß zumindest teilweise durch die maximale Intensität und/oder Gesamtdosis der Einwirkung einer oder mehrerer durchgeführter Vorbereitungsmaßnahme(n) bestimmt. Bezüglich der Bestimmung durch die Gesamtdosis können unterschiedliche Einwirkungen, die aus unterschiedlichen Vorbereitungsmaßnahmen resultieren, geeignet berücksichtigt werden. Bei der Bestimmung durch die maximale Intensität wird der Tatsache Rechnung getragen, daß eine bewußt gewählte Einwirkung, oder aber auch eine unbewußt auftretende Einwirkung (z.B. durch eine defekte Autoklave) bereits zu einer wesentlichen Beeinträchtigung des Kunststoffs des Teils der Kuppelvorrichtung führen kann. Auch eine solche, durch ausschließliches Zählen der durchgeführten Vorbereitungsmaßnahmen nicht erfaßbare Situation wird dem Benutzer so angezeigt.

In einer praktischen Ausführungsform bestimmt sich das vorbestimmte Maß zumindest teilweise nach der Anzahl n von durchgeführten Vorbereitungsmaßnahmen. Dies stellt ein einfaches Maß dar, das von der Anzeigeeinrichtung gewisserweise mit einer inneren Zähluhr gemessen wird.

Vorteilhaft ist die Anzahl n = 1. So kann der Benutzer mit bloßem Auge erkennen, wann immer eine weitere Vorbereitungsmaßnahme stattgefunden hat. Dies ist insbesondere dann zweckmäßig, wenn das Teil nur wenige Male verwendet werden soll, insbesondere wenn der erste Zustand der Neuzustand ist, und bei Erreichen des zweiten Zustandes bereits eine erhebliche Beeinträchtigung des Teils der Kuppelvorrichtung vorliegt. Insbesondere dann, wenn die Kuppelvorrichtung bereits zu oft benutzt worden ist, werden dem Benutzer mit jeder weiter durchgeführten Vorbereitungsmaßnahme neue Warnzeichen gesetzt.

Zweckmäßig ist die Anzahl n eine vorgegebene Höchstanzahl n_{H} von Vorbereitungsmaßnahmen, nach deren Überschreitung ein sachgerechtes Funktionieren des Teils nicht mehr sichergestellt ist. Diese Höchstanzahl wird typischerweise vom Hersteller der elektrischen Kuppelvorrichtung angegeben und beträgt typischerweise 50, 100, 200 oder 500, je nach Art der elektrischen Kuppelvorrichtung. In diesem Fall gibt der erste Zustand den Neuzustand an, und der zweite Zustand denjenigen, bei dem das Teil durch ein Neuteil ersetzt werden sollte.

Vorteilhaft weist die Anzeigeeinrichtung zur Erleichterung der Unterschiedserkennung eine Referenzanzeige auf, deren Erscheinungsbild durch die Einwirkung nicht veränderlich ist und zumindest teilweise in dem ersten und/oder zweiten Zustand vorliegt. Für den Benutzer ist die absolute Einschätzung eines farblichen Erscheinungsbilds schwieriger als der relative Vergleich zwischen zwei Erscheinungsbildern. Liegt das Erscheinungsbild der Referenzanzeige beispielsweise in dem das zu erfolgende Aussortieren angebenden Zustand vor, so kann der Benutzer sehr genau feststellen, wann er aussortieren muß, nämlich wenn das Erscheinungsbild des Sichtfeldes bzw. Sichtfeldabschnittes mit dem der Referenzanzeige übereinstimmt. So ist dem Benutzer die Möglichkeit gegeben, das Teil nicht zu spät, aber auch nicht zu früh auszutauschen. Umgekehrt kann einem Benutzer auch dadurch ein Austauschsignal gegeben werden, daß das farbliche Erscheinungsbild des Sichtfeldes bzw. Sichtfeldabschnitts sich von demjenigen der Referenzanzeige unterscheidet, wohingegen ursprünglich kein Unterschied bestand.

Besonders vorteilhaft weist die Anzeigeeinrichtung eine Skalenanzeige mit einem durch die Einwirkung nicht veränderlichen Erscheinungsbild auf, mittels dem im Vergleich mit dem aktuellen farblichen Erscheinungsbild des Sichtfeldes bzw. Sichtfeldabschnittes feststellbar ist, in welchem Maße die bisher erreichte Änderung bereits von dem farblichen Erscheinungsbild des ersten Zustands fortgeschritten ist und/oder welche Änderung noch zu dem des zweiten Zustands fehlt. Mit der Skalenanzeige wird die oben erwähnte innere Zähluhr der Anzeigeeinrichtung nach außen für den Benutzer sichtbar. Dabei kann auch eine numerische Anzeige als numerische Übersetzung der Farbskala vorgesehen sein. Auf diese Weise erhält der Benutzer zusätzlich hilfreiche Informationen, z.B. wann er etwa neue Teile der elektrischen Kuppelvorrichtung nachbestellen muß, weil die bisherigen bald auszutauschen sind.

Zusätzlich oder auch alternativ kann auch zweckmäßig vorgesehen sein, daß die sichtbare Änderung von dem farblichen Erscheinungsbild des ersten Zustandes in das des zweiten Zustandes schlagartig erfolgt, insbesondere bei Überschreitung einer Einwirkungsschwelle. Wenn der Benutzer an Zwischenzuständen nicht interessiert ist, wird ihm die für ihn relevante Information, nämlich ,"geht noch" bzw. "geht nicht mehr" in einer Form geliefert, die eine häufige Überprüfung der Anzeigeeinrichtung nicht mehr erfordert. Der Benutzer verwendet das Teil wieder, solange er beispielsweise ein grünes Erscheinungsbild wahrnimmt, und tauscht aus, sobald er z.B. erstmals ein rotes Erscheinungsbild wahrnimmt.

Dabei ist es zweckmäßig, wenn die Einwirkungsschwelle einer Anzahl n_{c} von Vorbereitungsmaßnahmen entspricht, wobei n_{c} im Bereich zwischen 80% der Höchstanzahl n_{H} und 100% von n_{H}, bevorzugt im Bereich zwischen 90% von n_{H} und 100% von n_{H} liegt und weiter bevorzugt gleich n_{H} ist. Insbesondere durch die bei unterschiedlichen Vorbereitungsmaßnahmen auftretenden unterschiedlichen Einwirkungen kann die Einwirkungsschwelle bereits vor dem Erreichen der Höchstanzahl von Vorbereitungsmaßnahmen erreicht werden. Bei einem möglicherweise unnötig früh durchgeführten Austausch des Teils wird der damit einhergehende Wertverlust jedenfalls eingeschränkt. Gleichzeitig wird ein Sicherheitspuffer bereitgestellt.

Zweckmäßig ist das Teil der elektrischen Kuppelvorrichtung ein Stecker, eine Buchse oder ein Kabel. Solange diese Teile als eine Kuppelvorrichtung vereint bleiben, ist es lediglich wichtig, daß jede Kuppelvorrichtung mindestens ein erfindungsgemäßes Teil mit der Anzeigeeinrichtung aufweist. Dabei ist daran gedacht, das Teil mit der Anzeigeeinrichtung zu versehen, bei dem das Sichtfeld gut erkennbar ist und einfach herstellbar ist. Es ist allerdings auch daran gedacht, mehrere Teile oder auch alle Teile einer elektrischen Kuppelvorrichtung mit einer Anzeigeeinrichtung auszustatten, was insbesondere von Vorteil sein kann, wenn mehrere gleichartige Kuppelvorrichtungen während des Gebrauchs durcheinander geraten.

Zweckmäßig gehören zu der/den Vorbereitungsmaßnahme(n) Reinigen, Sterilisieren und/oder Autoklavieren. Im allgemeinen wird sich die durchgeführte Vorbereitungsmaßnahme einsatzabhängig nach entsprechenden Sicherheitsrichtlinien richten. Diese Vorbereitungsmaßnahmen können zu unterschiedlichen Einwirkungen führen, wobei die des Autoklavierens zur Dekontamination am zuverlässigsten sind, und daher bevorzugt zu berücksichtigen sind.

Besonders vorteilhaft weist die Anzeigeeinrichtung einen Stoff auf, der auf Temperaturänderung, insbesondere Wärmezufuhr, Druckänderung, insbesondere Druckerhöhung, und/oder Feuchtigkeit durch Farbänderung reagiert. Dabei kann die Farbänderung bereits durch eine der angesprochenen Einwirkungen, aber auch beispielsweise erst durch die Kombination zweier oder mehrerer Einwirkungen erfolgen.

Insbesondere kann der Stoff ein Farbmittel oder Farbstoff, aber auch eine Kombination mehrerer Farbmittel oder Farbstoffe aufweisen. Dabei kann es sich sowohl um anorganische als auch um organische Farbmittel und sowohl um natürliche als auch synthetische Farbmittel handeln. Diese Farbmittel und/oder Farbstoffe sind selbst oder durch die Art ihrer Aufnahme in die Anzeigeeinrichtung derart beschaffen, daß sie eine der einer Einwirkung der Vorbereitungsmaßnahme zugeordneten "Echtheit" nicht aufweisen, sondern infolge der Einwirkung sich ändern. z.B. ausbluten.

Die Erfindung betrifft auch ein Verfahren zum Herstellen eines Teils einer elektrischen Kuppelvorrichtung, insbesondere der Medizintechnik und insbesondere eines oben beschriebenen Teils, wobei das Teil zumindest teilweise aus Kunststoff ist, wobei dem Kunststoff ein Stoff hinzugefügt wird, dessen farbliches Erscheinungsbild sich als Reaktion auf eine Einwirkung ändert, die aus einer Vorbereitungsmaßnahme für einen Einsatz des Teils resultiert.

Dabei ist insbesondere vorgesehen, den Stoff dem Kunststoff bereits in einem Verfahrensschritt beizumischen, bei dem die Formbildung des Teils noch nicht abgeschlossen ist. Der Stoff kann allerdings auch in einem späteren Verfahrensschritt auf den Kunststoff aufgebracht werden, bei dem das Teil bereits geformt ist. Insbesondere ist daran gedacht, den Stoff als Teil einer Anzeigeeinrichtung auf den Kunststoff in einem Verfahrensschritt aufzubringen, der sich an den letzten Herstellungsschritt eines Teils ohne Anzeigeeinrichtung anschließt.

Ein weitere Aspekt der Erfindung betrifft die Verwendung eines Stoffes, dessen farbliches Erscheinungsbild sich dadurch ändert, daß er einer Einwirkung ausgesetzt wird, die aus einer Vorbereitungsmaßnahme für den Einsatz eines Teils einer elektrischen Kuppelvorrichtung, insbesondere der Medizintechnik resultiert, in einem solchen Teil.

Weitere Vorteile und Merkmale der Erfindung ergeben sich aus der folgenden Beschreibung von einzelnen Ausführungsformen der Erfindung. Dabei wird auf die Figuren 1 bis 5 Bezug genommen, von denen
- Fig. 1: erfindungsgemäße Teile einer elektrischen Kuppelvorrichtung zeigen, nämlich Fig. 1a eine Buchse, Fig. 1b einen Stecker, Fig. 1c ein Kabel und Fig. 1d eine aus den Teilen zusammengesetzte elektrische Kuppelvorrichtung in einem gekuppelten Zustand zeigt, .
- Fig. 2: ein Beispiel für eine Anzeigeeinrichtung mit Sichtfeld zeigt, wobei in den Teilfiguren 2a, 2b und 2c unterschiedliche farbliche Erscheinungsbilder des Sichtfelds gezeigt sind,
- Fig. 3: eine weitere Ausgestaltung einer Anzeigeeinrichtung eines erfindungsgemäßen Teils zeigt,
- Fig. 4: noch eine weitere Anzeigeeinrichtung eines erfindungsgemäßen Teils zeigt, und
- Fig. 5: ein Diagramm einer Vorbereitungsmaßnahme für ein erfindungsgemäßes Teil zeigt.

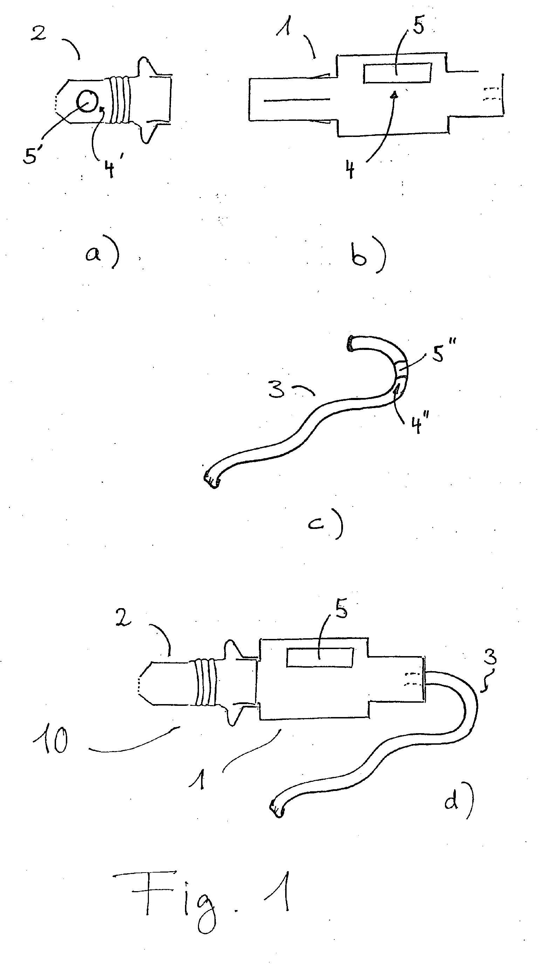

In Fig. 1a ist eine Buchse 2 einer elektrischen Kuppelvorrichtung 10 gezeigt, wobei letztere in zusammengesetztem Zustand in Fig. 1d dargestellt ist und beispielsweise als push-pull Steckmechanismus ausgebildet ist. Die Buchse 2 weist eine Anzeigeeinrichtung 4' auf, die ein gut erkennbares Sichtfeld 5' aufweist. Anhand des farblichen Erscheinungsbilds des Sichtfelds 5' kann der Benutzer der Buchse 2 erkennen, ob die Buchse 2 nach den bisher durchgeführten Vorbereitungsmaßnahmen für ihren Einsatz, z. B. in einem Operationssaal noch geeignet ist oder durch ein Neuteil ersetzt werden muß.

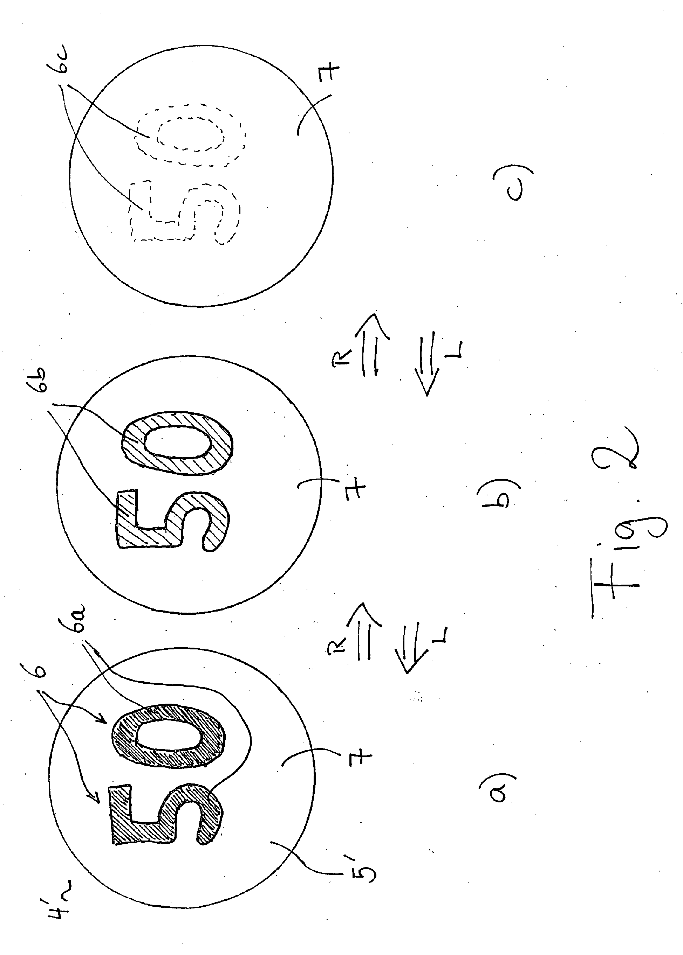

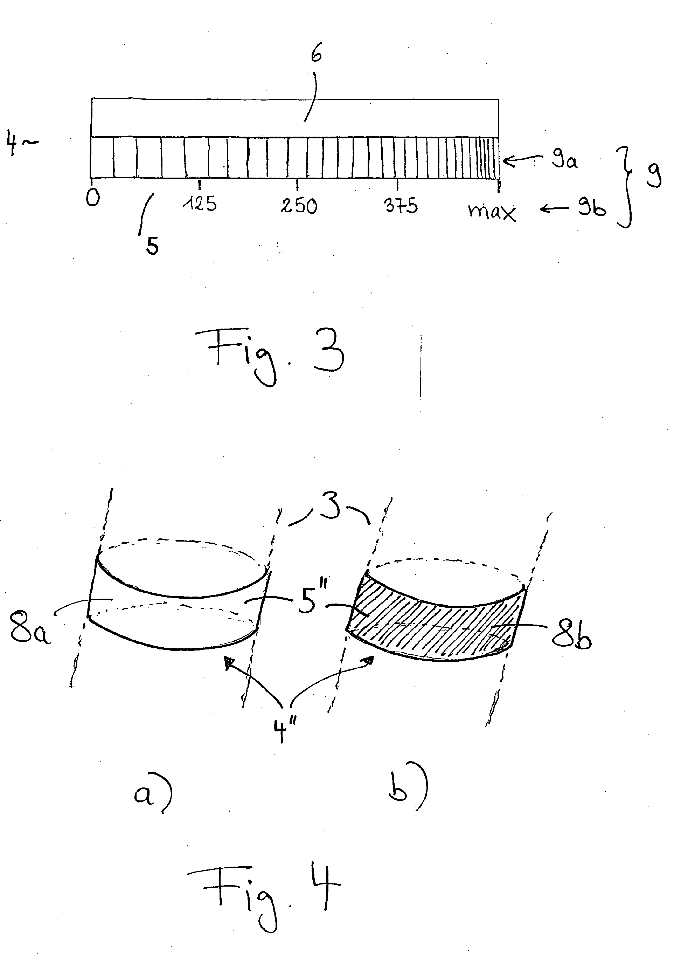

In Fig. 1b ist ein Stecker 1 gezeigt, der in die in Fig. 1a dargestellte Buchse 2 paßt und mit der Buchse 2 die Kuppelteile der in Fig. 1d dargestellten Kuppelvorrichtung 10 bildet. Der Stecker 1 weist eine Anzeigeeinrichtung 4 auf, die ein gut erkennbares Sichtfeld 5 aufweist, deren Zweck entsprechend den obigen Erläuterungen dem des Sichtfelds 5' entspricht, welches aber anders ausgebildet ist (vgl. auch die Figuren 2 und 3). In Fig. 1c ist schließlich ein Kabel 3 einer elektrischen Kuppelvorrichtung dargestellt, welches eine Anzeigeeinrichtung 4" aufweist, deren Sichtfeld 5" als ein Streifen ausgebildet ist, der das Kabel 3 umläuft.

In Fig. 1 d erkennt man, wie ein Stecker 1 mit einer Buchse 2 koppelt, wobei Buchse 2 und Stecker 1 Kabelanschlüsse aufweisen, wobei allerdings nur die Verkabelung des Steckers 1 mit dem Kabel 3 dargestellt ist. Bei der in Fig. 1d dargestellten Kuppelvorrichtung 10 weist nur der Stecker die Anzeigeeinrichtung mit dem Sichtfeld 5 auf, es wäre aber auch denkbar, zusätzlich oder alternativ die Buchse mit einer Anzeigeeinrichtung auszustatten, wie in Fig. 1a dargestellt, oder das Kabel, wie in Fig. 1 c dargestellt, oder auch an jedem der Teile eine Anzeigeeinrichtung mit Sichtfeld vorzusehen.

In Fig. 2 ist die Anzeigeeinrichtung 4' aus Fig. 1a vergrößert dargestellt, und zwar in drei unterschiedlichen Zuständen des Erscheinungsbildes von dessen Sichtfeld 5'. Für die durch die Zeitpfeile R dargestellte Variante stellt der in Fig. 2a dargestellte Zustand das farbliche Erscheinungsbild im Neuzustand dar, wie es an einem neuen Teil einer elektrischen Kuppelvorrichtung erkennbar ist. Im Sichtfeld 5' sind zwei unterschiedliche Abschnitte zu erkennen, nämlich ein sensitiver Abschnitt 6 in Form einer "50", und eine diesen sensitiven Abschnitt 6 umgebenden Referenzanzeige 7.

In diesem Beispiel erscheint die Referenzanzeige 7 in einem gelben Farbton, der autoklavierecht ist, sich also unter Einwirkung von Aufoklavierzyklen nicht verändert. Der sensitive Abschnitt 6 dagegen erscheint als grüner Farbton 6a, wird sich aber unter Einwirkung von z.B. Autoklavierzyklen verändern. In diesem Fall soll die dargestellte grüne 50 anzeigen, daß das entsprechende Teil der Kuppelvorrichtung nach 50 Autoklavierzyklen durch ein Neuteil ersetzt werden sollte.

Fig. 2c stellt das Sichtfeld 5' dar, nachdem die entsprechende Anzeigeeinrichtung 4' (zusammen mit dem dazugehörigen Teil der Kuppelvorrichtung) 50 Autoklavierzyklen ausgesetzt worden ist. Dabei hat sich das Erscheinungsbild des sensitiven Abschnittes 6 unter Einwirkung der Autoklavierzyklen derart geändert, daß es nun ebenfalls in einem gelben Farbton erscheint, der dem Farbton gelb der Referenzanzeige 7 gleich ist. Dies soll durch die gestrichelten Linien in Fig. 2c angedeutet sein, die den sensitiven Bereich 6 von der Referenzanzeige 7 andeutungsweise trennen. In Fig. 2c ist das tatsächliche Erscheinungsbild des Sichtfelds 5' gleichmäßig gelb. Für den Benutzer ist jede Spur der ursprünglich grünen 50 verschwunden. Dies ist für ihn das Signal, das entsprechende Teil der Kuppelvorrichtung bzw. die ganze Kuppelvorrichtung zu ersetzen.

In diesem Beispiel erfolgt die Änderung des farblichen Erscheinungsbilds des sensitiven Abschnitts 6 des Sichtfelds vom grünen Zustand 6a zum gelben Zustand 6c einigermaßen gleichmäßig, so daß der Benutzer auch mehrere Zwischenzustände noch gut unterscheiden kann. Ein Beispiel für einen solchen Zwischenzustand ist in Fig. 2b dargestellt, wo das Erscheinungsbild des sensitiven Abschnitts 6 nun ein gelbgrüner Mischton bzw. ein helles Grün 6b ist.

Es ist klar, daß anstatt der unterschiedlichen Farbtöne gelb und grün auch andere Farbtöne verwendet werden können, solange sichergestellt ist, daß das Erscheinungsbild des Endzustands 6c mit dem der Referenzanzeige 7 übereinstimmt. Dabei ist auch denkbar, daß die Zahl 50 als dunkler Grauton im Vergleich zu einem helleren Grauton der Referenzanzeige 7 zunächst deutlich erkennbar ist, und nach 50 Autoklavierzyklen den gleichen Grauton wie die Referenzanzeige hat. Insbesondere kann die Referenzanzeige 7 durch die Kunststoffaußenhülle des Teils der Kuppelvorrichtung selbst gebildet sein.

Bei einer anderen Ausgestaltung der Erfindung ändert sich das farbliche Erscheinungsbild der Anzeigeeinrichtung 4' entsprechend den Zeitpfeilen L. Zunächst hat das ganze Sichtfeld 5' ein einheitliches Erscheinungsbild. Erst nach einigen Autoklavierzyklen wird eine Änderung des sensitiven Abschnitts 6 erkennbar, dessen Erscheinungsbild z.B. wiederum über eine Zwischenstufe 6b zu einem Erscheinungsbild 6a geändert wird, welches sich deutlich vom Erscheinungsbild der Referenzanzeige 7 unterscheidet. In diesem Fall wird die Warnwirkung dadurch erhöht, daß das sich (nach 50 Vorbereitungszyklen) ergebende Erscheinungsbild 6a des sensitiven Abschnitts 6 in Rot gestaltet wird.

Nach diesem eben vorgestellten Prinzip sind der Farb- und Formgestaltung nur wenig Grenzen gesetzt, so kann z.B. statt der Zahl 50 ein anderes Symbol oder eine andere Zahl verwendet werden, die z.B. eine andere Maximalanzahl an Vorbereitungsmaßnahmen angibt.

In Fig. 3 ist eine weitere Ausgestaltung einer Anzeigeeinrichtung 4 mit Sichtfeld 5 dargestellt. In diesem Fall sind im Sichtfeld 5 ein sensitiver Abschnitt 6 und eine Skalenanzeige 9 zu erkennen. Wie bereits in obiger Beschreibung deutlich gemacht wurde, verändert sich ein anfängliches Erscheinungsbild des sensitiven Abschnitts 6 aufgrund durchgeführter Autoklavierzyklen derart, daß es beispielsweise nach hier n_{H} gleich 500 Autoklavierzyklen in einem Farbton erscheint, der vom ursprünglichen Farbton mit bloßem Auge zu unterscheiden ist. Selbstverständlich können auch Zwischenzustände bereits mit bloßem Auge unterscheidbar sein, wie es insbesondere bei dieser Ausführungsform der Fall ist.

Die Skalenanzeige 9 weist eine Farbskala 9a auf, die an eine numerische Skala 9b derart gekoppelt ist, daß sie Zahlenwerten jeweils farbliche Erscheinungsbilder zuordnet, die jeweils dem farblichen Erscheinungsbild entsprechen, das der sensitive Abschnitt 6 nach einer entsprechenden Anzahl von Autoklavierzyklen hat. Die Farbskala 9a setzt sich aus diesen Farben zusammen.

Der Benutzer kann daher aus dem in Fig. 3 dargestellten Sichtfeld 5 der Anzeigeeinrichtung 4 leicht durch Abgleich des aktuellen Erscheinungsbilds des sensitiven Abschnitts 6 mit dem dazu passenden Skalenbereich der Farbskala 9a mit Hilfe der numerischen Skala 9b die Anzahl der bisher durchgeführten Autoklavierzyklen ablesen. Damit weiß er u.a., wie oft er das entsprechende Teil der elektrischen Kuppelvorrichtung bzw. die gesamte Kuppelvorrichtung noch verwenden kann, bevor die Herstellerangabe (hier n_{H} = 500) erreicht ist. Er kann sich somit jederzeit einen zuverlässigen Überblick über das Restverwendungspotential seiner elektrischen Kuppelvorrichtungen verschaffen, und somit rechtzeitig vor dem Überschreiten der Höchstzahl 500 Nachschub bestellen.

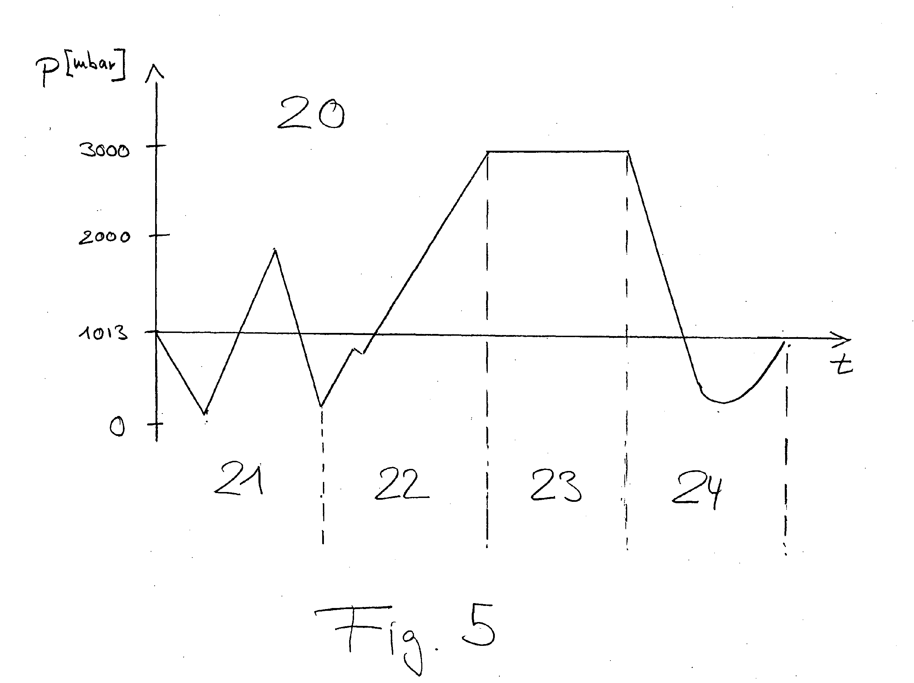

In Fig. 4 ist eine nochmals weitere Ausgestaltung einer Anzeigeeinrichtung 4" dargestellt, deren Sichtfeld 5" das Teil der Kuppelvorrichtung umläuft, welche in diesem Beispiel das in Fig. 1c Kabel 3 ist. Die Wirkungsweise dieser Anzeigeeinrichtung ist etwas anders als diejenige der oben beschriebenen Anzeigeeinrichtungen, indem sich nämlich das farbige Erscheinungsbild 8a des Sichtfelds 5 zwar mit jedem durchgeführten Autoklavierzyklus ändert, aber nur minimal, so daß mit bloßem Auge zunächst kaum ein Unterschied gegenüber dem ursprünglichen Erscheinungsbild 8a festzustellen ist. Die sichtbare Änderung vom farblichen Erscheinungsbild 8a in das farbliche Erscheinungsbild 8b, welches in Fig. 4b gezeigt ist, erfolgt schlagartig nach Überschreiten einer Einwirkungsschwelle, die in diesem Beispiel genau der maximalen Autoklavieranzahl 100 entspricht, auf das das entsprechende Teil der Kuppelvorrichtung bzw. die gesamte Kuppelvorrichtung ausgelegt ist.

In diesem Beispiel ist das farbliche Erscheinungsbild 8b sehr dunkel, während das farbliche Erscheinungsbild 8a sehr hell ist; und beispielsweise dem farblichen Erscheinungsbild des Kabels 3 entspricht. Die Anzeigeeinrichtung 4" ist derart am Kabel 3 angeordnet, daß bei jeder Verkabelung und Kuppelherstellung der Kuppelvorrichtung dem Benutzer sofort auffällt, wenn das Erscheinungsbild des umlaufenden streifenförmigen Sichtfeldes 5" dunkel geworden ist. Damit weiß er, daß er das Kabel 3 bzw. die gesamte elektrische Kuppelvorrichtung ersetzen sollte.

Darüber hinaus kann daran gedacht werden, das Sichtfeld 5" so zu gestalten, daß der Übergang zum farblichen Erscheinungsbild 8b bereits einige Autoklavierzyklen vor der tatsächlichen Herstellerangabe von 100 Autoklavierzyklen annimmt, z.B. nach 80 oder 90 Autoklavierzyklen. Auf diese Weise wird ein intrinsischer Sicherheitspuffer der Differenz von Autoklavierzyklen, d.h. in diesem Beispiel 20 oder 10 Autoklavierzyklen, eingebaut. Wird die Kuppelvorrichtung noch nach dem Erscheinen des dunklen Erscheinungsbilds 8b des Sichtfelds 5" einige Male weiter verwendet, so befindet man sich dennoch im sicheren Bereich, d.h. ein sachgerechtes Funktionieren der Kuppelvorrichtung ist noch gewährleistet, obwohl das Erscheinungsbild des Sichtfeldes 5" sich bereits verändert hat. Wiederum sind mehrere Wandlungen der Farb- und Formgestaltung möglich. Beispielsweise kann nach einer bestimmten Einwirkungsschwelle ein z.B. roter Schriftzug erscheinen, der den Benutzer darauf hinweist, daß er das Teil nicht mehr verwenden darf.

In Fig. 5 ist schließlich noch der zeitliche Druckverlauf dargestellt, der bei einem typischen Autoklavierzyklus in einer Autoklave erreicht wird. Danach werden in einer ersten Entlüftungsphase 21 zunächst eine erste Unterdruckvorphase, und danach mit einem darauffolgenden Druckanstieg mit nach folgendem Druckabbau eine zweite Unterdruckvorphase durchlaufen. In einer Druckaufbauphase 22 wird der Druck bis auf einen Maximaldruck von etwa 3 bar erhöht, wobei in dieser Phase die Temperatur bereits mit erhöht wird, so daß sie auf ca. 134°C ansteigt. In der eigentlichen Sterilisationsphase 23 herrscht diese Temperatur etwa 5 Minuten, und anschließend noch 15 Minuten eine Temperatur von 121 °C, wobei während der gesamten Sterilisationsphase 23 der Druck auf etwa 3 bar gehalten wird. Schließlich wird in der Trocknungsphase 24 der Druck abgebaut und eine Untardrucknachphase durchlaufen, in der getrocknet und anschließend wieder belüftet wird. Bevorzugt reagiert der in der Anzeigeeinrichtung für den sensitiven Abschnitt verwendeten Stoff bei einer Temperatur und/oder bei einem Druck, die/der während der Sterilisationsphase 23 in der Autoklave herrscht/herrschen.

Die in den Figuren dargestellten Ausführungsformen stellen nur einige wenige beispielhafte Möglichkeiten dar, ein erfindungsgemäßes Teil einer elektrischen Kuppelvorrichtung zu realisieren. Demgemäß können die in der obigen Beschreibung sowie in den Ansprüchen offenbarten Merkmale der Erfindung sowohl einzeln als auch in beliebiger Kombination für die Verwirklichung der Erfindung in ihren verschiedenen Ausführungsformen wesentlich sein.

## Patentansprüche

1. Teil (1, 2, 3) einer elektrischen Kuppelvorrichtung (10), insbesondere zum Einsatz in der Medizintechnik, mit einer Anzeigeeinrichtung (4) zum Anzeigen einer aus einer Vorbereitungsmaßnahme für einen Einsatz des Teils resultierenden Einwirkung auf das Teil, **dadurch gekennzeichnet, daß** die Anzeigeeinrichtung ein Sichtfeld (5) aufweist, dessen farbliches Erscheinungsbild sich als Reaktion auf die aus der Vorbereitungsmaßnahme resultierende Einwirkung zumindest abschnittsweise ändert.

2. Teil nach Anspruch 1, **dadurch gekennzeichnet, daß** die Änderung des farblichen Erscheinungsbilds von einem ersten Zustand (6a) in einen zweiten Zustand (6b) erfolgt, deren Unterschied mit bloßem Auge erkennbar ist, wenn die Einwirkung ein vorbestimmtes Maß übersteigt.

3. Teil nach Anspruch 2, **dadurch gekennzeichnet, daß** in dem ersten und in dem zweiten Zustand unterschiedliche Farbtöne erscheinen.

4. Teil nach Anspruch 2 oder 3, **dadurch gekennzeichnet, daß** in dem ersten und in dem zweiten Zustand unterschiedliche Graustufen erscheinen.

5. Teil nach einem der Ansprüche 2 bis 4, **dadurch gekennzeichnet, daß** sich das vorbestimmte Maß zumindest teilweise durch die maximale Intensität und/oder Gesamtdosis der Einwirkung einer oder mehrerer durchgeführter Vorbereitungsmaßnahme(n) bestimmt.

6. Teil nach einem der Ansprüche 2 bis 5, **dadurch gekennzeichnet, daß** sich das vorbestimmte Maß zumindest teilweise nach der Anzahl n von durchgeführten Vorbereitungsmaßnahmen bestimmt.

7. Teil nach Anspruch 6, **dadurch gekennzeichnet, daß** die Anzahl n = 1 ist.

8. Teil nach Anspruch 6, **dadurch gekennzeichnet, daß** die Anzahl n eine vorgegebene Höchstanzahl n_{H} von Vorbereitungsmaßnahmen, vorzugsweise 50, 100, 200 oder 500 ist, nach deren Überschreitung ein sachgerechtes Funktionieren des Teils nicht mehr sichergestellt ist.

9. Teil nach einem der Ansprüche 2 bis 8, **dadurch gekennzeichnet, daß** die Anzeigeeinrichtung zur Erleichterung der Unterschiedserkennung eine Referenzanzeige (7, 9) aufweist, deren Erscheinungsbild durch die Einwirkung nicht veränderlich ist und zumindest teilweise in dem ersten und/oder zweiten Zustand vorliegt.

10. Teil nach einem der Ansprüche 2 bis 9, **dadurch gekennzeichnet, daß** die Anzeigeeinrichtung eine Skalenanzeige (9) mit einem durch die Einwirkung nicht veränderlichen Erscheinungsbild aufweist, mittels dessen im Vergleich mit dem aktuellen farblichen Erscheinungsbild des Sichtfeldes bzw. Sichtfeldabschnittes (6) feststellbar ist, in welchem Maße die bisher erreichte Änderung bereits von dem farblichen Erscheinungsbild des ersten Zustands fortgeschritten ist und/oder welche Änderung noch zu dem des zweiten Zustands fehlt.

11. Teil nach einem der Ansprüche 2 bis 10, **dadurch gekennzeichnet, daß** die sichtbare Änderung von dem farblichen Erscheinungsbild des ersten Zustandes in das des zweiten Zustandes schlagartig erfolgt, insbesondere bei Überschreitung einer Einwirkungsschwelle.

12. Teil nach Anspruch 11, mittelbar oder unmittelbar zurückbezogen auf Anspruch 8, **dadurch gekennzeichnet, daß** die Einwirkungsschwelle einer Anzahl n_{c} von Vorbereitungsmaßnahmen entspricht, wobei n_{c} im Bereich zwischen 80% der Höchstanzahl n_{H} und 100% von n_{H}, bevorzugt im Bereich zwischen 90% von n_{H} und 100% von n_{H} liegt und weiter bevorzugt gleich n_{H} ist.

13. Teil nach einem der vorhergehenden Ansprüche, **dadurch gekennzeichnet, daß** es ein Stecker (1), eine Buchse (2) oder ein Kabel (3) ist.

14. Teil nach einem der vorhergehenden Ansprüche, **dadurch gekennzeichnet, daß** zu der/den Vorbereitungsmaßnahme(n) Reinigen, Sterilisieren und/oder Autoklavieren (20) gehört/gehören.

15. Teil nach einem der vorhergehenden Ansprüche, bei dem die Anzeigeeinrichtung einen Stoff aufweist, der auf Temperaturänderung, insbesondere Wärmezufuhr, Druckänderung, insbesondere Druckerhöhung, und/oder Feuchtigkeit durch Farbänderung reagiert.

16. Verfahren zum Herstellen eines Teils einer elektrischen Kuppelvorrichtung, insbesondere für medizinische Zwecke und insbesondere nach einem der vorhergehenden Ansprüche, wobei das Teil zumindest teilweise aus Kunststoff ist, **dadurch gekennzeichnet, daß** dem Kunststoff ein Stoff hinzugefügt wird, dessen farbliches Erscheinungsbild sich als Reaktion auf eine Einwirkung ändert, die aus einer Vorbereitungsmaßnahme für einen Einsatz des Teils resultiert.

17. Verwendung eines Stoffes, dessen farbliches Erscheinungsbild sich **dadurch** ändert, daß er einer Einwirkung ausgesetzt wird, die aus einer Vorbereitungsmaßnahme für den Einsatz eines Teils einer elektrischen Kuppelvorrichtung, insbesondere für medizinische Zwecke, resultiert, in einem solchen Teil.
